# EUROPEAN PATENT SPECIFICATION

(11) **EP 2 126 609 B1**
(45) Date of publication and mention of the grant of the patent: **27.03.2013**
(21) Application number: 08701945.1
(22) Date of filing: 25.01.2008
(51) Int. Cl.: G06T 7/40, G06T 7/00, G01T 1/29

(54) **TOOLS FOR AIDING IN THE DIAGNOSIS OF NEURODEGENERATIVE DISEASES**
WERKZEUG ZUR UNTERSTÜTZUNG BEI DER DIAGNOSTIZIERUNG NEURODEGENERATIVER ERKRANKUNGEN
INSTRUMENTS D'AIDE AU DIAGNOSTIC DE MALADIES NEURODEGENERATIVES

(30) Priority: 30.01.2007 US 887163 P
(43) Date of publication of application: 02.12.2009
(73) Proprietor: GE Healthcare Limited, Little Chalfont Buckinghamshire HP7 9NA (GB)
(72) Inventor: LILJA, Johan, Axel, S-75109 Uppsala (SE); THURFJELL, Nils, Lennart, S-75109 Uppsala (SE)
(74) Representative: Canning, Lewis R.
(86) International application number: PCT/GB2008/000272
(87) International publication number: WO 2008/093057

(56) References cited:
- WO-A-02/16333
- WO-A-2006/014382
- US-A1- 2006 074 290
- KEMPPAINEN N M ET AL: "Voxel-based analysis of PET amyloid ligand [11C]PIB uptake in Alzheimer disease." NEUROLOGY 14 NOV 2006, vol. 67, no. 9, 14 November 2006 (2006-11-14), pages 1575-1580, XP002477395 ISSN: 1526-632X

## Description

### Field

The present invention relates to tools for aiding in the diagnosis of neurodegenerative diseases. In particular, the present invention relates to apparatus and methods for applying image analysis techniques to brain image data for aiding in the diagnosis of neurodegenerative diseases, such as, for example, Alzheimer's disease (AD).

### Background

Various neurodegenerative diseases, such as, for example, Alzheimer's disease are known to be difficult to diagnose definitively *in vivo.* For example, although it is possible to identify subjects who may have a genetic predisposition to the development of AD [1,2], often it is only possible to provide a provisional diagnosis based upon data derived from laboratory, clinical and late stage neuro-imaging studies when various characteristic symptoms become apparent to the skilled clinician.

Various techniques have been used to aid in the preparation of such a provisional diagnosis for AD. These techniques include various physical screening tests, such as, for example, the optical test devised by Newman [3] in which an optical technique is used to determine whether a patient's eye has sustained ganglion cell loss consistent with the advance of AD.

Such provisional diagnoses are useful. However, recently, increasing evidence has emerged that the pathological process of AD may begin decades prior even to the possibility of any such provisional diagnosis being made, in the so-called preclinical stage of the disease. This preclinical stage may be divided into two main phases, namely: an initial "latent phase" in which no observable symptoms are present and a subsequent "prodromal phase" in which mild symptoms insufficient for provisional clinical diagnosis are present.

Various attempts have therefore also been made to try to provide earlier stage diagnosis by attempting to identify various signs of the pathological process during the two preclinical phases. To date, two main techniques have been used to identify any abnormal variations that might be associated with early stage pathology of AD, namely: a) magnetic resonance imaging (MRI) and functional magnetic resonance imaging (fMRI) of the brain [4,7]; and b) evaluation of metabolic changes in the brain by monitoring the uptake of radioactive ¹⁸F-2-fluro-2-deoxy-D-glucose (FDG) by using a positron emission tomography (PET) scanner [2,5,6].

Whilst such techniques do help in the diagnosis of AD, there is a limitation in that all of the abovementioned methods measure secondary effects of the disease and there is a need to provide an improved way of rapidly and accurately assessing patients for detection of the pathological process of AD, in all three of the preclinical, provisionally diagnosed and diagnosed phases of the disease. This is particularly important in the preclinical stage, where early identification of the disease process and treatment is advisable for preventing or slowing the advance of the disease. Moreover, there also exists a need for a way better to assess the progress of AD, including any response to treatment, in those patients in any of the three disease phases.

US2006074290 A1 discloses a method to acquire imaging and non-imaging datasets from like objects. A linkage is found using a partial least squares (PLS) technique between imaging and non-imaging datasets. The linkage is then reduced to an expression of a single numerical assessment. The single numerical assessment is then used as an objective, quantified assessment of the differences and similarities between the objects. The data each dataset can be aspects of performance, physical characteristics, or measurements of appearance.

### Summary of the invention

Various aspects and embodiments of the present invention have been developed to provide tools to aid in the diagnosis and monitoring of neurodegenerative diseases (such as, for example, AD) with the aforementioned disadvantages of conventional techniques borne in mind.

According to a first aspect of the present invention, there is provided a system for clinical evaluation of neurodegenerative disease present in a subject. The system comprises an image acquisition module that is operable to acquire image data representative of a brain of a subject and an image analyser. The image analyser is operable to determine a quantitative value from the image data, wherein the quantitative value is indicative of the level of neurodegenerative disease present in the brain of the subject.

According to a second aspect of the present invention, there is provided a method for clinical evaluation of neurodegenerative disease present in a subject. The method comprises acquiring image data representative of a brain of a subject and analysing the image data to determine a quantitative value from the image data. The quantitative value is indicative of the level of neurodegenerative disease present in the brain of the subject.

Various embodiments of systems and methods according to these aspects of the present invention have the advantage that the quantitative value represents a precise value (e.g. a numerical value) that can be used by various healthcare professionals to aid in their diagnoses. The quantitative value can thus be used to measure whether or not various indicators for particular neurodegenerative diseases are present, as well as to provide an indication of any disease severity. Moreover, because such a value is quantitative, it can also be used to aid healthcare professionals in tracking any changes in the condition of a patient over various time periods, thereby ensuring that such systems, methods and computer program products find use in helping to monitor the progress of any disease (e.g. deterioration/remission), the effectiveness of any treatments administered, etc.

### Brief description of the drawings

Various aspects and embodiments of the present invention will now be described with reference to the accompanying drawings, in which:
Figure 1 shows a system for clinical evaluation of neurodegenerative disease present in a subject according to an embodiment of the present invention;
Figure 2 shows a method for aiding clinical evaluation of neurodegenerative disease present in a subject according to various embodiments of the present invention;
Figure 3 shows a workflow comprising various methods according to aspects of the present invention;
Figure 4 shows anatomic standardization of PET data using a single subject MRI template that has been smoothed using an anisotropic filter;
Figure 5 shows the extraction of diagnostic features using a grey/white matter ratio according an aspect of the present invention;
Figure 6a shows use of an intensity profile as a diagnostic feature from an image taken from a subject with AD according to an aspect of the present invention;
Figure 6b shows use of an intensity profile as a diagnostic feature from an image taken from a normal control (NC) subject according to an aspect of the present invention;
Figure 7a shows a three dimensional (3D) graphical display of results for a brain volume of interest (VOI) and grey/white matter measurements derived in accordance with an aspect of the present invention;
Figure 7b shows a graphical display of results for a brain intensity profile analysis derived in accordance with an aspect of the present invention;
Figure 7c shows a graphical display of results for a brain voxel based features analysis derived in accordance with an aspect of the present invention;
Figure 8a shows a 3D graphical display of results for a brain intensity profile analysis derived in accordance with an aspect of the present invention for a subject with AD; and
Figure 8b shows a 3D graphical display of results for a brain intensity profile analysis derived in accordance with an aspect of the present invention for a normal subject without AD.

### Detailed description

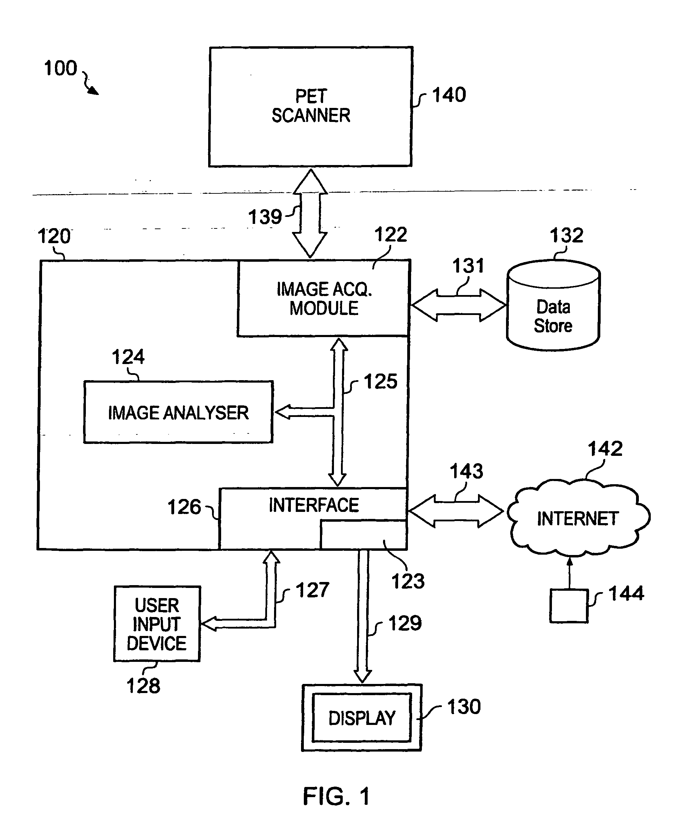

Figure 1 shows a system 100 for clinical evaluation of neurodegenerative disease present in a subject according to an embodiment of the present invention. The system 100 includes a data processing apparatus 120 that is configured to provide various interfaces 123,126, an image acquisition module 122 and an image analyser 124. The interfaces 123,126, image acquisition module 122 and image analyser 124 can be logically coupled together by way of a data bus 125 under the control of a central processing unit (not shown).

The data processing apparatus 120 provides a first general purpose interface 126 for interfacing the data processing apparatus 120 to external components. In this embodiment the external components include: an input data link 127 coupled to a user input device 128 (e.g. a mouse/keyboard/etc.), a network data link 143 coupled to the Internet 142, and a display data link 129 coupled to a display 130. Additionally, the general purpose interface 126 also provides a graphical user interface (GUI) 123 through which a user of the system 100 can input data, commands etc., and receive visual information by viewing the display 130.

The GUI 123 may be operable to generate a two- and/or three-dimensional representation of at least part of the brain of the subject. Such representations may include colour coding of regions according to uptake of a substance in the brain in respective of those regions. This provides ease of visualisation for users of the system 100. In addition, in various embodiments, a user can also rotate images and/or slice 3D images by manipulating the GUI 123 using the input device 128.

The GUI 123 can also be further operable to link data in tabular form with the three dimensional representation. For example, a user might click data values in a displayed table and corresponding region in an image of the brain light up, or vice versa. This enables the user to rapidly access quantitative values from a displayed image.

In various embodiments, the data processing apparatus 120 can be provided by a general purpose computer, such as, for example a personal computer (PC). Such a general purpose computer can use software modules to provide both the image acquisition module 122 and the image analyser 124, and hence can be implemented by upgrading the functional capability of existing equipment using software upgrades. For example, a computer program product 144, comprising computer code, may be transmitted from a remote server (not shown) via the Internet 142 to the data processing apparatus 120 through the network data link 143.

The system 100 also comprises an optional positron emission tomography (PET) scanner 140 coupled to the data processing apparatus 120 by a data link 139, and an optional data store 132 coupled to the data processing apparatus 120 by a data link 131. The PET scanner 140 and/or the data store 132 may be configured to provide image data to the image acquisition module 122. For example, where no PET scanner is provided, image data could be provided from the data store 132 that may contain previously generated image data stored therein. Such previously generated image data could be generated remotely from the system 100 (e.g. in a remote hospital, etc. where suitable image data generation facilities are available), and subsequently transferred to the data store 132 from where it can be retrieved by the image acquisition module 122. The image acquisition module 122 is further operable to transfer image data generated by the PET scanner 140 to the data store 132 for archiving purposes.

The image analyser 124 is operable to determine a quantitative value from the image data, wherein the quantitative value is indicative of the level of neurodegenerative disease present in the brain of the subject. The quantitative value can be a numerical figure that is determined on the basis of the presence of various anatomical and/or chemical variations from a set of normal image data. In a preferred mode of operation, the image analyser 124 uses image data from the PET scanner 140 to determine a quantitative value from the image data by determining the concentration of amyloid plaques in the brain of the subject. Imaging of the concentration of amyloid plaques in the human brain is one promising technique for obtaining measures that are directly coupled to the disease process in AD and methods for quantitative evaluation of amyloid imaging data are hence important.

Determination of the amyloid content, e.g. β-amyloid, is particularly important for diagnosis of AD and for monitoring the effect of therapy. Several radioactive tracers for imaging of amyloid content using PET or SPECT are under development and one aspect of the present invention is related to automated analysis of such data. Moreover, this aspect of the present technique can also reduce the complexity of the data acquisition allowing for a simplified protocol to be used, and hence reduce the time needed to obtain a quantitative value indicative of the level of any neurodegenerative disease by avoiding the longer acquisition times required for multiple imaging techniques.

Whilst the system 100 preferably operates using at least one mode that detects the presence of amyloid for analysing brain amyloid content, it is to be understood that the system 100 need not necessarily be limited to this mode of operation. For example, various modes of operation of the system 100 could combine one or more of: PET imaging of amyloid content, FDG imaging of brain metabolism, MRI, fMRI, etc. Such modes, particularly when combined, may be used to obtain more accurate imaging and thus more accurate quantitative value metrics, for example, at the expense of image data acquisition time, image data processing time, etc., according to any particular desired clinical application of the system 100.

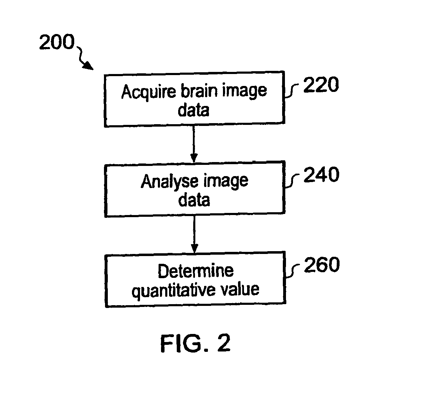

Figure 2 shows an embodiment of a method 200 for aiding clinical evaluation of neurodegenerative disease present in a subject. The method 200 may be implemented using various embodiments of apparatus made in accordance with the present invention, such as, for example, the system 100 illustrated in Figure 1.

The method 200 comprises acquiring brain image data 220. This step may itself further comprise merely obtaining the image data, e.g. from a data storage device, or may comprise performing a PET scan of at least part of the brain of the subject. In the latter case, a radioactive tracer substance may be administered to a patient. For example, the radioactive tracer substance might comprise a chemical entity that selectively binds to amyloid protein, such as radiopharmaceuticals like the GE® Pittsburgh Compound B (PiB) family of tracers as described in WO 02/16333 and WO2004/083195, or the tracer FDDNP and analogues as described in WO 00/10614. Accordingly, a quantitative value may be determined from the amyloid concentration in the brain of the subject that is useful for aiding in the diagnosis of neurodegenerative disease.

The method 200 comprises the steps of analysing image data 240 and determining the quantitative value 260. The combination of these two steps 240,260 may comprise a variety of techniques, several examples of which are described in more detail below. For example, analysing image data 240 may comprise one or more of: defining reference regions in an image defined by the image data, determining the quantitative value from uptake of a substance as a ratio of an uptake in grey brain matter to an uptake in white brain matter, determining the quantitative value as a rate of change in the image data magnitude along a predetermined projection in the brain, etc.

In various embodiments of the method 200, provision is made to make maximum use of the available image information for the analysis of a subject's PET scan. For example, if an MRI scan is available, the anatomic standardisation can be driven by the MRI scan. If PET/CT data is available the CT component can be used. This scheme helps ensure that the maximum accuracy is achieved given the information available at the time of the analysis. Such a method is described in more detail below.

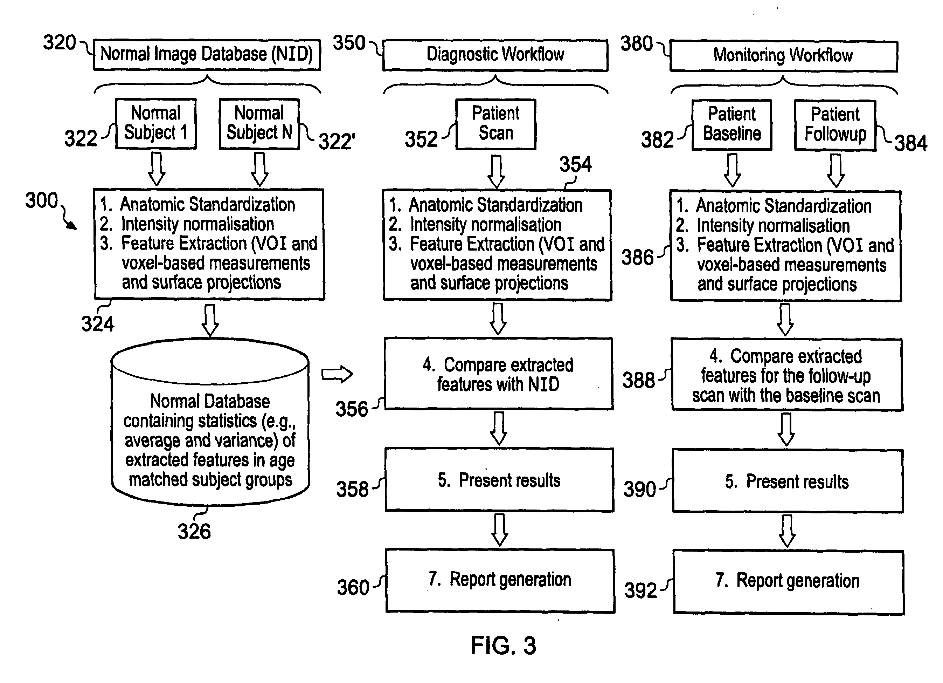

Figure 3 shows a workflow 300 comprising various methods 320,350,380 according to certain aspects and embodiments of the present invention. For example, one or more of the methods 320,350,380 may be implemented by the system 100 of Figure 1 or be included as part of the method 200 of Figure 2.

A first aspect of the workflow 300 provides a method 320 for obtaining a normal image database (NID). The N)D is used to provide a set of control data that can subsequently be used to identify abnormal physiological, chemical or anatomical data indicators that may indicate the presence of neurodegenerative disease. The NID may be obtained once, e.g. at a central medical facility, and distributed, or might be provided locally at one or more systems based upon testing normal subjects.

In various embodiments of the invention, the NID can be constantly updated by scanning normal subjects at a particular location to improve the accuracy of the data therein. Such NID data might also be shared across many systems provided at different locations, so as to improve the global overall accuracy of the data in the NID at all of the locations. This is particularly useful where, for example, a small hospital is provided with a system according to an embodiment of the invention but which by itself may not have a sufficient number of subjects/patients using it to be able to provide a locally derived statistically useful NID data set.

Scans from a large number of normal subjects can be processed and included in the NID at steps 322, 322'. Only two scans 322, 322' are shown for clarity, but clearly N may correspond to any positive integer, with N preferably being as large as practically possible so as to obtain an optimised NID.

At step 324, processing of the image data obtained from the scans at steps 322, 322' is performed. Processing of the image data consists of three steps: 1) anatomic standardisation; 2) intensity normalisation; and 3) feature extraction. Various ways of implementing these three processes are described below, beneath respective headings.

### Anatomic standardization

The purpose of anatomic standardization (also sometimes referred to as spatial normalisation) is to transform data from different subjects into a standard anatomical space, such as, for example, the Talairach and MNI (Montreal Neurological Institute) space. Anatomic standardisation is achieved by applying a spatial transformation to one image set (which may be referred to as the floating image) so that it matches the second image set (which may be referred to as the reference image). Most methods proceed by iteratively adjusting the transformation so as to maximise some similarity measure computed between the transformed floating image and the corresponding reference image. Finding a suitable transformation usually involves the use of an optimisation algorithm. The type of transformation and the number of parameters that are used determine the accuracy of the anatomic standardisation. In general anatomic standardisation using high resolution anatomic images (e.g. MRI) can be performed with higher accuracy than when using functional images such as PET and SPECT.

Anatomic standardization allows for a direct comparison of data from different subjects, since a specific anatomical structure occupies the same location in the standardized space. Anatomical standardization also, for example, allows for the creation of the normal image database and for the use of a volume of interest (VOI) template for automated quantification of the uptake in different regions, for example, where various tracers are administered to a subject.

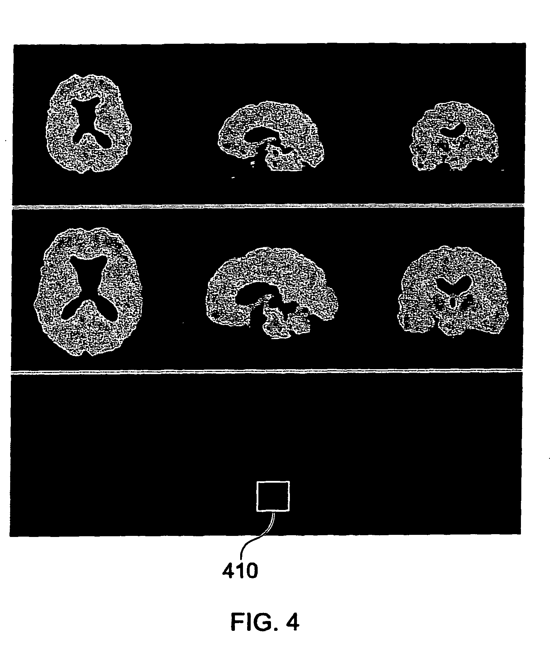

Accurate anatomical standardization is important and in general the process is more accurate where anatomical images are used in conjunction with PET derived imaging data. However, since anatomical images are not always be available, it is important to have a method that can perform accurate anatomical standardization directly using the PET data. Because of this, a method can be employed for automatically selecting an image analysis mode depending on the class/classes of the image data, as mentioned previously. Such selection techniques may include:
a) Where a subject's MRI data is available, the subject's MRI is co-registered with the PET scan. The MRI image is spatially normalised by employing a non-rigid registration that maximises the similarity between the subject's MRI and an MRI template in the standardized space. This results in a transformation that maps the MRI to standardized space. The transformation obtained in the previous step is then used to transform the PET scan to standardised space.
b) Where no MRI data is available, but a PET image data has been obtained using a combined PET and computer tomography (CT) scanner (PET/CT), the image data from the CT scan and the PET scan would normally already be in registration. However, this assumption is checked and if the image data is not aligned, the CT image data is co-registered with the PET image data. The CT component of the PET/CT image data is spatially normalised by employing a non-rigid registration that maximises the similarity between the subjects' CT and a template in the standardized space. This results in a transformation that maps the CT data to standardized space. The transformation obtained in the previous step is then used to transform the PET scan image data to standardised space.
c) Where only PET image data is available, the PET scan image data is spatially normalised by employing a non-rigid registration that maximises the similarity between the PET data and a template in the standardized space. For amyloid data (e.g. PIB) there is a characteristic difference in image pattern when comparing images from an Alzheimer's subject with that of a normal control. This may lead to systematic errors if a standard method for anatomic standardisation of PET data is employed. Moreover, for amyloid data, it is especially important to have good registration of the area around the reference region (see below). To overcome these difficulties, the following method is employed. As a reference template, a single subject MRI brain registered to the MNI space is used (see Fig. 4, for example). To reduce the risk of getting local minima in the similarity function, the reference template is filtered with an anisotropic filter that makes the template smoother while still preserving tissue boundaries.

For the registration of a PET scan to the reference template, a similarity function based on normalised mutual information is used. Moreover, the registration is performed in two steps. In the first step, the PET scan is globally registered to the reference template using a polynomial transformation with 18 parameters. In the second step, a local registration around the reference region is performed. A bounding box defined by an inner and an outer 3D shape (box, sphere or irregular) is placed around the reference region and a local registration of the data within the inner shape is performed using a rigid transformation. Data in the area between the inner and the outer shape is interpolated in order to ensure a smooth transition between the data inside and outside the bounding box. This method ensures good overall registration of the subject's brain with an increased accuracy of data in the vicinity of the reference region (i.e. inside the bounding box). Figure 4 shows a bounding box 410 for a reference region around Pons, but it is understood that other reference regions can be used (e.g. the Cerebellum).

It is noted that the template used in procedure b) may be MRI or CT based, while the template used in step c) is MRI based. The registration method used can be a method based on the maximisation of a similarity measure including correlation, mutual information, normalised mutual information and the transformation used to spatially normalise the data including, for example, affine, polynomial, discrete cosine transformation (DCT), etc.

### Intensity normalisation (reference region)

In order to allow for a comparison of image data across various subjects, the data can be intensity scaled to account for injected activity, different subjects' weight etc. One technique that may be employed is to scale data according to the uptake in a region that is supposed to be unaffected by whatever neurodegenerative disease of interest is being investigated.

In various embodiments of the present invention, a reference region in an image defined by the image data from which to determine the level of neurodegenerative disease present in the subject is defined. The reference region may, for example, correspond to a sub-area of the brain, such as, for example: Pons, thalamus, cerebellum, etc. The use of such a relatively small reference area increases the need to have a robust definition of this area.

For example, for amyloid imaging (e.g. using C11-PIB) the region used may be the grey matter areas of the cerebellum. Typically, this region is manually outlined in co-registered MRI. However, for an automated method, the reference region must be defined in standardised space. To make this step robust, a maximum probability mask can also be applied. In the description that follows, examples are described as to: a) how such a mask can be created; and b) how the mask can be used.
a) Creation of a reference region probability mask: the maximum probability grey matter mask was created according to the following technique: 1) for N subjects, image data was co-registered between MRI and a PET scan; 2) an expert was used to outline the cerebellum reference region in the co-registered MRI data; 3) all data was transformed to standardised space using the method outlined in the previous section; and 4) a probability map was computed that, for each voxel, showed the probability of that voxel being present in all reference regions of the N subjects. Hence, a voxel that was part of the reference region in all subjects was given a numerical value of 1.0, a voxel that was part of all except one was given a value (N-1)/N, and so on. The same approach was also applied to other regions in order to create probability reference masks for reference regions such as, for example, Pons and sub-cortical white matter.
b) Using the reference region probability mask to intensity normalise data: the mask was applied using the following technique: 1) the probability mask was applied to the anatomical standardized PET image data; 2) the average voxel value of all voxels defined by the mask was computed, and the voxels were given the relative contribution according to the corresponding probability in the mask; 3) the whole image was divided with the computed average. A ratio image was thus obtained, whereby the tracer uptake was scaled relative to the reference region.

It is noted that the use of such a probability mask in combination with an anatomic standardisation technique that has high accuracy in the area around the reference region allows for a robust extraction of a reference value, and hence increases the accuracy in the comparisons across scans.

### Feature extraction

The purpose of feature extraction is to extract information that is characteristic of a particular neurodegenerative disease that is being investigated. Different measurements (or features) are complimentary, and can be used to provide diagnostic information, to provide accurate measurements for longitudinal follow-up, and also to enhance visual interpretation of various image data.

Various techniques may be used to identify features of interest, four examples of which will now be described in more detail:
a) VOIs can be applied to the data in order to determine target region to reference region ratios. One way to do this is by: 1) applying a VOI atlas to the ratio image (i.e. an anatomically standardized and intensity normalized scan), wherein the VOI atlas includes definitions of anatomical regions such as brain lobes, Brodmann areas etc.; and 2) computing statistics within the different VOI's defined by the atlas. The VOI atlas can stored in different formats including a labelled volume or polygons. Then there must exist a mapping from the atlas to the standardized space. In its simplest form, this is a one-to-one mapping so each voxel in the labelled volume corresponds to a voxel in the standardized space. Any structure in the VOI atlas can then be used as a VOI which can be applied to an image in standardised space, and different properties of the voxels within the VOI can be computed such as the mean, variance and standard deviation of all voxel values defined by the VOI.
b) It is noted that PET amyloid data exhibit characteristically different patterns depending on whether the subject has Alzheimer's disease or not. A PET amyloid scan of an AD patient shows high signal in the cortical areas, whereas a healthy subject shows high signal in white matter regions and low signal in the cortical regions. Because of this, a VOI-based feature that is particularly useful for analysis of amyloid data is the use of a grey-white matter ratio. This may be obtained by: 1) applying a VOI atlas to the ratio image (i.e. an anatomically standardized and intensity normalized scan), wherein the VOI atlas includes definitions of anatomical regions such as brain lobes, Brodmann areas etc., and where in addition, the atlas defines grey matter and white matter areas of the brain; 2) for VOI's such as brain lobes, computing the uptake in the grey matter region but only considering voxels defined by the VOI and the grey matter mask; 3) for the same VOI's, computing the uptake in the white matter region but only considering voxels defined by the VOI and the white matter mask; and 4) computing the grey-white ratios for each VOI.

In various embodiments, a quantitative value is determined as a ratio of a substance uptake in grey brain matter to the substance uptake in white brain matter. The substance can, for example, be any whose ratio changes when neurodegenerative disease is present: e.g. FDG for PET imaging, amyloid tracers with PET imaging, etc. One advantage of such a technique is that it means that reference area normalisation is not necessarily needed.

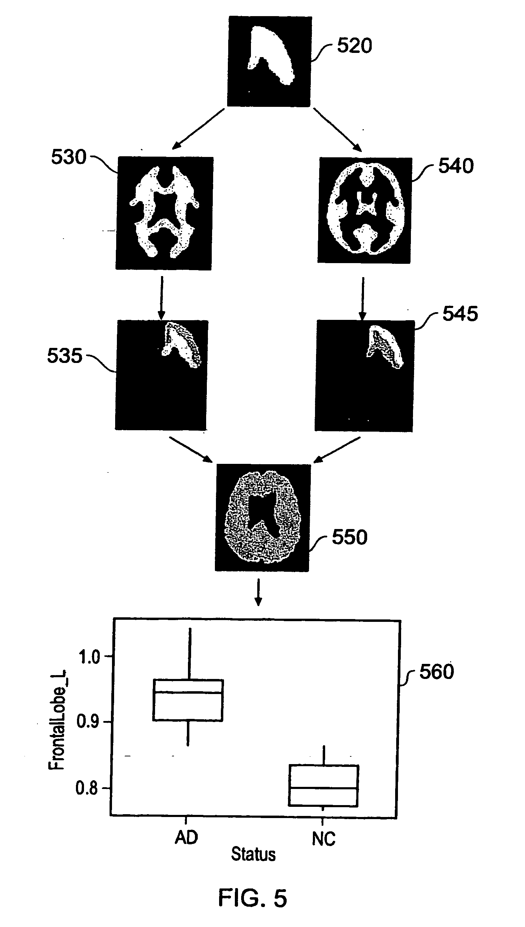

One way of using such a grey/white matter ratio is described below in more detail in connection with Figure 5.
c) Intensity profile features can be used. One way to do this is by: 1) defining a number of surface points and rays along a surface normal in the standardized space, and a label defining to which VOI in the VOI atlas (i.e. which anatomical region) each surface point belongs; 2) using the ratio image (i.e. the anatomically standardized and intensity normalized scan) to calculate intensity profiles for the predefined VOI's using traces perpendicular to the brain surface (see, for example, Figures 5a and 5b); 3) computing a property describing the intensity distribution along each ray (one such property is the gradient describing the rate of change in intensity along each ray); and 4) averaging the computed property (e.g. the gradient values) for all rays within each VOI into one number that can be used to define a quantitative value indicative of the level ofneurodegenerative disease present in the brain of the subject.

In various embodiments, the quantitative value is determined as a rate of change in the image data magnitude along a predetermined projection in the brain. This allows determination of whether neurodegenerative disease is present, and its quantification for subsequent studies/tests/scans on the subject.

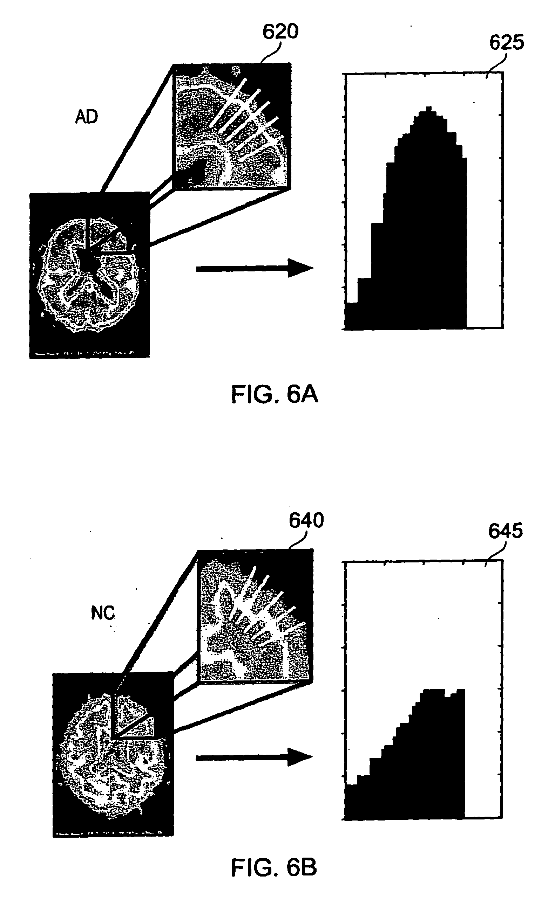

One way of using such intensity profile features is described below in more detail in connection with Figures 6a and 6b.
d) Voxel based features can be used. One way to do this is by using the voxel intensity in the whole brain, or as masked by anatomical regions defined by the VOI atlas, as diagnostic and monitoring features.
e) For amyloid data, it is desirable to combine the computed features into one "Amyloid index". This can be done by computing a weighted average of the VOI values as computed by VOI analysis and/or intensity profile analysis and dividing with the corresponding value in one or several reference regions.

Having determined image data for the NID, in accordance with one or more of the techniques referred to above, the normal image data is then stored in a database 326, along with various statistical information, such as, for example, averages and variances of extracted features for age matched subject groups.

Figure 3 also shows a second aspect of the workflow 300. This aspect of the workflow 300 provides a method 350 for clinical evaluation of neurodegenerative disease present in a subject, by way of determining a quantitative value from image data that is indicative of the level of any neurodegenerative disease present in the brain of the subject.

The method 350 comprises performing a scan of a subject/patient 352. The scan may be one or more of a PET scan, MRI scan, CT scan etc. In one preferred mode of operation, the scan comprises a PET scan of the amyloid content of the patient's brain. The image data from the scan(s) is processed at step 354 to extract clinically relevant information. For example, the processing of step 354 may provide a quantitative value from the image data. At step 356, the result of the processing of step 354 is compared to that of a normal subject to determine whether or not any abnormalities indicative of neurodegenerative disease are present in the brain of the subject. The results of this comparison are presented at step 358, and then a report is generated at step 360.

In the illustrated example, the processing of step 354 can use any of the techniques referred to above in connection with the processing of step 324 used for providing the NID. However, those skilled in the art will recognise that aspects and embodiments of the present invention need not be so limited.

Various ways to compare the extracted features of the scan 352 with the NID are possible at step 356. One way is by comparison of various diagnostic features. For example: a) VOI features can be used in which the mean value within different VOI's is compared to the normal range as defined by the NID and deviations including Z-scores are computed; b) the grey/white matter ratio can be used in which the ratios for the different VOI's are compared to the normal range as defined by the NID and deviations including Z-scores are computed; c) intensity profile features can be used in which the values corresponding to intensity properties along each ray (max intensity, max gradient and other features) are compared to the normal range as defined by the NID and deviations including Z-scores are computed; and/or d) voxel based features can be used in which the voxel data is compared to average and standard deviation data in the NID, the Z-score images are computed and a cluster analysis method is then applied to the data and all clusters below a certain size are discarded.

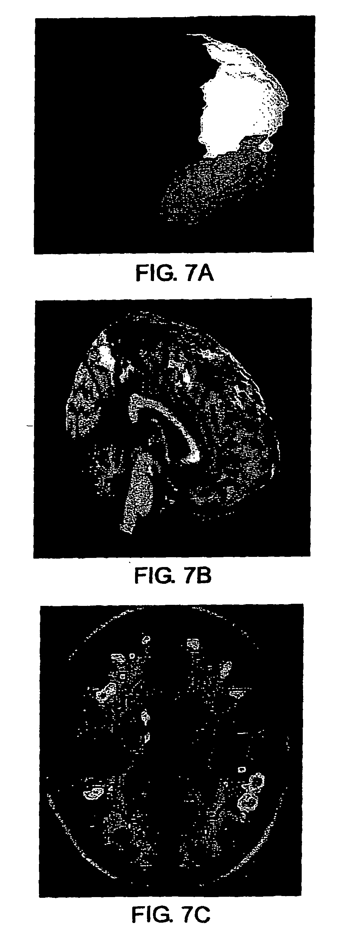

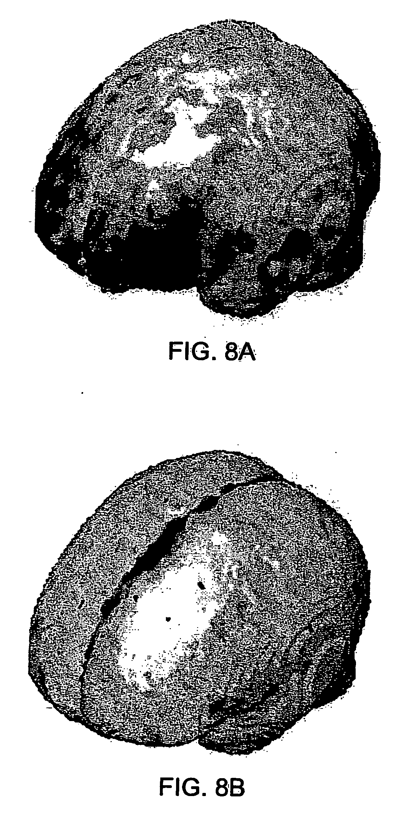

Once the scan has been compared to the NID at step 356, the result may then be presented at step 358. Figures 7a-7c and 8a and 8b, below, show various examples, in both two- and three-dimensions, of how this may be achieved. Of course, such results might be better presented in colour to further enhance the presence of any deviations of the scan from a normal subject.

In one embodiment, where VOI features or grey/white ratio are used, data is presented in tables and graphically with a surface rendering of a brain image in standardised space with VOI definitions outlined. The VOI's and/or grey/white matter are colour coded according to significance. For intensity profile features, the data can be presented as surface projections with the value along each ray (max intensity, max gradient and other features) (Figs. 8a and 8b), and/or the z-score data of intensity profile features compared to normal data projected in 2D slices and on a 3D rendering of a brain in standardized space (Figs. 7a-7c). For voxel based features, deviation images and z-score maps can also be displayed superimposed on MRI data.

In this embodiment, report generation 360 is also provided. This can be archived for future use and/or transmitted to a remote location (hospital etc.) for study by interested personnel. The report may contain the following information: a) patient information, date etc.; b) images showing the original patient scan; c) processed images showing the results; d) tables with measurements (e.g. the VOI results); and a statement indicating whether the findings of an investigation lie within a normal range or not.

Figure 3 also shows a third aspect of the workflow 300. This aspect of the workflow 300 provides a method 380 for monitoring the progress of any neurodegenerative disease present in a subject.

The method 380 comprises performing a follow-up scan of a subject/patient 384 having previously performed a patient baseline scan 382. The scans may be one or more of a PET scan, MRI scan, CT scan etc. In one preferred mode of operation, the scan comprises a PET scan of the amyloid content of the patient's brain.

Similarly to the diagnostic scan 350, the image data from the scan(s) is/are processed at step 386 to extract clinically relevant information. For example, the processing of step 386 may provide a quantitative value from the image data. At step 388, the result of the processing of step 386 is compared to the results of the previous baseline scan 382 to quantify the progress of neurodegenerative disease present in the brain of the subject. The results of this comparison are presented at step 390, and then a report is generated at step 392. The way the results are presented 390 and the report generated 392 may be similar to the steps 358 and 360 for the diagnostic workflow, respectively, as referred to above.

In the illustrated example, the processing of step 386 can use any of the techniques referred to above in connection with the processing of step 324 used for providing the NID. However, those skilled in the art will recognise that aspects and embodiments of the present invention need not be so limited.

In the comparison step 388, VOI features can be used in which the mean value within the different VOI's for the follow-up scan is compared to the corresponding values in the baseline scan. Differences can then be computed and compared to the normal range as defined by the NID. The grey/white ratio can also be used where the ratios for the different VOI's for the follow-up scan are compared to the corresponding values in the baseline scan. Differences can then be computed and compared to the normal range as defined by the NID. Intensity profile features can be used where the value along each ray (max intensity, max gradient and other features) for the follow-up scan is compared to the corresponding values in the baseline scan. Differences can then be computed and compared to the normal range as defined by the NID. Voxel based features can also be used where difference images and statistical parametric maps showing increases and decreases are computed.

Figure 4 shows the registration of a PET amyloid scan. The reference image (lower row) is a single subject MRI scan defined in MNI space. The MRI scan has been blurred with an anisotropic filter that smoothes data within a tissue class but preserves boundaries between tissues. This Figure shows PET data before (upper row) and after (middle row) anatomic standardisation. Figure 4 also illustrates the use of a bounding box 410 used in a two step registration.

Figure 5 shows the extraction of diagnostic features using a grey/white matter ratio according an aspect of the present invention. In the standard space used (e.g. the MNI space) a number of anatomical regions define volumes of interests (VOIs) as well as a white matter mask 530 and a gray matter mask 540. The specific VOI shown in Figure 5 corresponds to the Frontal Lobe 520.

The quantification works as follows: the Frontal Lobe VOI 520 is combined with the white matter mask 530 using a logical AND to produce a VOI covering only the white matter region of the frontal lobe 535. This new VOI is applied to the image data 550 and is used to extract the values in the white matter in the frontal lobe. Similarly, the Frontal Lobe VOI 520 is combined with the grey matter mask 540 using a logical AND to produce a VOI covering only the grey matter region of the frontal lobe 535. This VOI is applied to the image data 550 and is used to extract the values in the gray matter in the frontal lobe. These two values are then combined so as to produce a Frontal Lobe Grey/White matter ratio, and the box plot 560 shows how this ratio can separate between Alzheimer subjects (AD) and normal controls (NC). It is clear to those skilled in the art that the grey and white matter regions need not necessarily be computed according to the aforementioned procedure, for example, the grey and white matter VOIs could equally well have been drawn interactively so as to produce a grey and a white matter VOI.

Figure 6a shows use of an intensity profile as a diagnostic feature from an image taken from a subject with AD according to an aspect of the present invention.

Figure 6b shows use of an intensity profile as a diagnostic feature from an image taken from a normal control (NC) subject according to an aspect of the present invention.

This embodiment of the method works as follows: a standard brain in the standardised space (e.g. MNI) is used and all voxels facing the background are considered as surface voxels. For all the surface voxels, the coordinate (x,y,z) along with the surface normal at that point is stored in a list denoted as the surface points list. Furthermore, there are label lists associated with the surface points list indicating to which anatomical region a certain surface point belongs. That is, each entry in the surface points list has a corresponding entry in the label list indicating which anatomical region the surface point belongs to. Having multiple lists allows multiple belongings (a surface point can, e.g., belong both to the left frontal gyrus and to the left frontal lobe).

When an image is analysed, it must first be spatially normalised so the surface points defined by the coordinates in the surface points list correspond to points on the surface of the brain in the image being analysed. Then the list of surface points is stepped through and for each point, a ray 620, 640 along the negative direction of the surface normal is computed. The ray is traversed starting at the surface point (or even slightly outside to make the method more robust) and the values along the ray are recorded and stored in an array while following the ray into the brain. The data is subsampled and the distance between each point along the ray, as well as the maximum distance into the brain, are parameters that can be changed. This procedure, will for each surface point, yield an intensity profile 625, 645 along the ray stored in the array. The intensity profile is then analysed and diagnostic features are extracted. In one instance of the invention, the maximum gradient computed as a difference between two points at a certain fixed distance is used as a diagnostic feature. In a second instance of the invention, the maximum intensity along the ray is computed. In a third instance of the invention, the ratio between points at a certain distance along the ray is computed. Of course, those skilled in the art will recognise that various other properties of the intensity profile can also be computed.

Figure 7a shows a three dimensional (3D) graphical display of results for a brain volume of interest (VOI) and grey/white matter measurements derived in accordance with an aspect of the present invention.

The 3D rendered brain is colour coded according to the analysis results obtained with the selected mode of operation. In one aspect of the invention, intensity profile features such as the max gradient are averaged over a certain brain structure (e.g. frontal lobe) and the computed average is then compared with the NID so as to produce a z-score. The z-scores obtained for the different brain structures may then be used to colour code the 3D rendered brain. For this, an appropriate colour scale is used so as to highlight areas with significant changes from normality as determined by the NID.

Figure 7b shows a graphical display of results for a brain intensity profile analysis derived in accordance with an aspect of the present invention. In this aspect of the invention, intensity profile features are not averaged across brain regions. Instead, each surface point is compared to the NID so as to produce a z-score, and this z-score can be used to colour code a generic MR template in standardised space. A user can set a cut-off value and only z-scores above the threshold will be displayed. For example, when the default cut-off is 2.0, a value must be at least two standard deviations away from the mean in order to be displayed. In order to allow the user to inspect the interior cortical areas as well, the standard brain is divided into two hemispheres and surface points are also defined on the interior surfaces.

Figure 7c shows a graphical display of results for a brain voxel based features analysis derived in accordance with an aspect of the present invention. This Figure shows results from a voxel-based analysis.

Figure 8a shows a 3D graphical display of results for a brain intensity profile analysis derived in accordance with an aspect of the present invention for a subject with AD. The intensity profiles are computed as described above, i.e. the list of surface points is stepped through and for each point, a ray along the negative direction of the surface normal is computed and the ray is traversed. The data along the ray is sub-sampled and an intensity profile is computed. Based on the intensity profile, different features can be computed such as the max value and the max gradient along the profile. The value at each surface point is then used to colour code the corresponding point on the 3D rendered brain using a pre-defined colour scale that can be changed by the user. This figure shows an AD brain analysed with the Max Gradient method.

Figure 8b shows a 3D graphical display of results for a brain intensity profile analysis derived in accordance with an aspect of the present invention for a normal subject without AD. The principles driving this display are the same as those described above in connection with Figure 8a.

Various embodiments of the invention may be implemented using one or more of: hardware, software and/or firmware. In one embodiment, computer code may be provided as a software product that is operable to upgrade an existing conventional system so as to provide new functionality in accordance with various aspects and/or embodiments of the present invention. The computer code may also, or additionally, be provided as a computer program product that may, for example, be provided on a carrier medium. Such a carrier medium may, for example, include signals transmissible over various links, for example, the Internet, a wireless link, an optical link, a radio link, an electronic link, a dedicated data/telephone link, LAN/WAN, etc., and may be used for upgrading an existing system, and/or the carrier medium may include computer code on a conventional carrier medium, such as a magnetic disk, a magnetic tape, an optical disk, a semiconductor device, etc.

Those skilled in the art will recognise that various embodiments may be used to upgrade existing systems. They would also realise that certain embodiments could be implemented using a distributed system, with different functions being performed by different data processing apparatus. For example, in various embodiments an image acquisition module may be incorporated in a PET scanner and/or a data processing apparatus could be a part of a PET scanner.

Whilst the present invention has been described in connection with various embodiments, those skilled in the art will realise that the invention is not limited to such embodiments and that many variations can be envisaged that fall within the scope of the invention as defined by the appended claims.

### References

1. US 2003/0233197, Carlos E. Padilla and Valeri I. Karlov
2. US 2005/0283054, Eric M. Reiman
3. US 2005/0094099, Richard W. Newman and Corinn C. Fahrenkrug
4. US 2005/0197560, Stephen M. Rao and Catherine L. Elsinger
5. US 2005/0215889, James C. Patterson II
6. US 2005/0273007, Ziad Burbar
7. WO 02/101407, Nicholas Fox and Ilya Charles
8. WO 2006/083378, Vladimir Kepe et al

## Claims

1. A positron emission tomography ("PET") method (200) for aiding clinical evaluation of neurodegenerative disease present in a subject, the method comprising:
acquiring PET (image data (220) representative of a brain of a subject; and
analysing the image data (240) to determine a quantitative value (260) from the image data, the quantitative value being indicative of a level of neurodegenerative disease present in the brain of the subject that is determined as the amyloid concentration in the brain of the subject;
wherein an anatomical standardisation process is performed to transform the image data into a standardized anatomical space using a two-step method, the two-step method comprising:
performing a global registration of the image data to a reference template; and
refining the registration by using a rigid registration in an area bounded by an inner and outer 3D shape, wherein the final registration is a combination of the global and rigid registrations and wherein the image data between the inner and outer 3D shape is interpolated so as to generate a smooth transition between a locally refined area and global registered data;
the PET method further comprising the steps of
defining a number of surface points and rays along the brain surface normal in the standardized anatomical space, and a label defining to which a volume of interest ("VOI") in a VOI atlas each surface point belongs;
calculating an intensity profile for each of the VOI's defined by the defining step using traces perpendicular to the brain surface from a scan that has been both anatomically standardized and intensity normalized, wherein the intensity normalization scales data according to uptake in a region that is supposed to be unaffected by the neurodegenerative disease; computing a property describing the intensity profile along each ray; and
averaging the computed property for all rays within each VOI into one number defining the quantitative value indicative of the level of neurodegenerative disease present in the brain of the subject.

2. The method (200) of Claim 1, wherein the image data depicts a signal from a, radioactive tracer substance comprising a chemical entity that selectively binds to amyloid protein, and said radioactive tracer substance had been previously administered to said subject.

3. The method (200) according to Claim 1 or Claim 2, comprising performing anatomical standardisation on amyloid PET data, wherein a reference template is obtained from a single-subject MRI scan in standardised space and wherein mutual information or normalised mutual information is used for optimisation.

4. The method (200) according to claim 3, wherein the single-subject MRI reference template is blurred with an anisotropic filter in order to preserve tissue boundaries.

5. The method according to Claim 1, wherein the step of analysing the image data (240) further comprises using a probability mask for defining reference regions in an image defined by the image data.

6. The method (200) of claim 5, wherein said probability mask is a grey matter mask (540).

7. The method (200) of claim 6, wherein said using step further comprises the step of creating said probability grey matter mask, said creating step further comprising the steps of:
co-registering image data between an MRI scan and a PET scan for a number of subjects;
outlining a reference region in the co-registered MRI data, wherein said outlining is performed by an expert;
transforming the image data to standardised space; and
computing a probability map that, for each voxel, shows the probability of that voxel being present in all of the reference regions of the subjects.

8. The method (200) of claim 7, wherein said outlining step is applied to at least one of the cerebellum, the Pons, and the sub-cortical white matter.

9. The method (200) according to claim 5, further comprising the step of using the reference region probability mask to intensity normalise data by
applying the probability mask to the anatomical standardized PET image data;
computing the average voxel value of all of the voxels defined by the mask, wherein the voxels are given the relative contribution according to the corresponding probability in the mask; and
dividing the whole image with the computed average from the computing step.

10. The method (200) of claim 2, further comprising the step of obtaining a ratio image whereby a tracer uptake is scaled relative a reference region.

11. The method (200) of claim 10, wherein the quantitative value is determined as a ratio of substance uptake in grey brain matter to the substance uptake in white brain matter, wherein the substance whose ratio changes when neurodegenerative disease is present.

12. The method (200) of claim 1, wherein the step of analysing the image data (240) further comprises the steps of:
applying a VOI atlas to the ratio image, wherein the VOI atlas includes definitions of anatomical regions, wherein said VOI atlas further defines grey matter and white matter areas of the anatomical regions;
computing the uptake in grey matter region in the voxels defined by the VOI and the grey matter mask, and computing, for the same VOI's; the uptake in the white matter region in the voxels defined by the VOI and the white matter mask; and
computing the grey-white ratios for each VOI.

13. The method (200) of claim 1, wherein the calculating step further comprises sampling the image data along the ray going from brain surface into the brain;
analysing the intensity profile and extracting a diagnostic feature comprising one of the maximum gradient computed as a difference between two points along the ray at a certain fixed distance and the ratio between points at a certain distance along the ray.

14. The method (200) according to Claim 1, further comprising the step of automatically selecting an anatomical standardisation and/or image analysis mode depending on the class/classes of the image data.

15. The method (200) of any one of Claims 1 to 14, further comprising computing an amyloid index as a weighted average of the values computed within a set of anatomical regions divided by the corresponding value in at least one reference region, where the value in each region is computed using VOI analysis and/or intensity profile analysis.

16. The method (200) of any one of Claims 1 to 14, further comprising generating a three dimensional representation of the brain of the subject, wherein the three dimensional representation includes colour coding of regions according to uptake of a substance in the brain in respective of those regions.

17. The method (200) of any one of Claims 1 to 14, further comprising generating a report, the report including one or more of: an indication of the presence or absence of neurodegenerative disease present in the subject; the quantitative value; a quantitative indication of the presence or absence of neurodegenerative disease present in the subject; patient information; date; time; images of an original patient scan; processed images of the results of the method according to any one of Claims 1 to 14; tables with measurements; VOI results; an amyloid index; and a statement of whether or not any findings lie within a normal parameter range.

18. A computer program product (144) comprising computer code operable to configure a data processing apparatus (120) for implementing one or more of the steps of the method (200) according to any one of Claims 1 to 17.

19. The computer program product (144) of Claim 18, provided on a carrier medium (142).

20. The computer program product (144) of Claim 19, wherein the carrier medium (142) comprises one or more of: a magnetic disk, a magnetic tape, an optical disk, an electronic signal, an optical signal, a radio signal, and a semiconductor device.

21. A system (100) for clinical evaluation of neurodegenerative disease present in a subject, the system comprising:
an PET image acquisition module (122) operable to acquire image data representative of a brain of a subject; and
an image analyser (124), wherein the image analyser is operable to perform the method of claim 1.

22. The system (100) according to claim 19, wherein the image analyser (124) is further operable to determine the quantitative value from the image data by determining the concentration of amyloid plaques in the brain of the subject.

23. The system (100) according to any of claims 21 and 22, wherein the image analyser (124) is further operable to define a reference region in an image defined by the image data from which to determine the level of neurodegenerative disease present in the subject.

24. The system (100) according to any of claims 21 to 23, wherein the image analyser (124) is further operable to determine the quantitative value as a ratio of a substance uptake in grey brain matter to the substance uptake in white brain matter.

25. The system (100) according to any of claims 21 to 24, wherein the image analyser (124) is further operable to determine the quantitative value as a rate of change in the image data intensity along a predetermined projection in the brain.

26. The system (100) according to any of claims 21 to 25, wherein the image analyser (124) is further operable automatically to select an anatomical standardisation and/or image analysis mode depending on the class/classes of the image data.

27. The system (100) according to any of claims 21 to 26, further comprising a graphical user interface (GUI) (123) operable to generate a three dimensional representation of the brain of the subject, wherein the three dimensional representation includes colour coding of regions according to uptake of a substance in the brain in respective of those regions.

28. The system (100) according to claim 27, wherein the GUI (123) is further operable to link data in tabular form with the three dimensional representation.

## Patentansprüche

1. Positronenemissionstomographie-("PET")-Verfahren (200) zur Unterstützung der klinischen Evaluierung einer bei einem Patienten bzw. Probanden vorliegenden neurodegenerativen Erkrankung, wobei das Verfahren umfasst:
Erfassen von PET-Bilddaten (220), die repräsentativ für ein Gehirn eines Patienten bzw. Probanden sind; und
Analysieren der Bilddaten (240), um einen quantitativen Wert aus den Bilddaten zu bestimmen (260), wobei der quantitative Wert ein Indikator für einen Grad der im Gehirn des Patienten bzw. Probanden vorliegenden neurodegenerativen Erkrankung ist, welcher quantitative Wert als die Amyloidkonzentration im Gehirn des Patienten bzw. Probanden bestimmt wird;
wobei ein Prozess anatomischer Standardisierung durchgeführt wird, um die Bilddaten in einen standardisierten anatomischen Raum unter Anwendung eines zweistufigen Verfahrens umzuwandeln, wobei das zweistufige Verfahren umfasst:
Durchführen einer globalen Registrierung der Bilddaten mit einer Bezugsvorlage; und
Verfeinern der Registrierung durch Verwenden einer rigiden Registrierung in einem durch eine innere und eine äußere 3D-Form begrenzten Bereich, wobei die endgültige Registrierung eine Kombination aus der globalen und der rigiden Registrierung ist und wobei die Bilddaten zwischen der inneren und der äußeren 3D-Form interpoliert werden, um einen sanften Übergang zwischen einem lokal verfeinerten Bereich und global registrierten Daten zu erzeugen;
wobei das PET-Verfahren weiterhin die Schritte umfasst
des Festlegens einer Anzahl von Oberflächenpunkten und Strahlen entlang der im standardisierten anatomischen Raum normalen Gehirnoberfläche, und einer Markierung, die festlegt, zu welchem Volumen von Interesse ("VOI") in einem VOI-Atlas jeder Oberflächenpunkt gehört;
des Berechnens eines Intensitätsprofils für jedes der durch den Festlegungsschritt festgelegten VOIs unter Verwendung von zur Gehirnoberfläche perpendikularen Spuren aus einem Scan, der sowohl anatomisch standardisiert als auch intensitätsnormalisiert worden ist, wobei die Intensitätsnormalisierung Daten in Übereinstimmung mit der Aufnahme in einer Region skaliert, von der angenommen wird, dass sie unbeeinträchtigt von der neurodegenerativen Erkrankung ist;
des Berechnens einer Eigenschaft, die das Intensitätsprofil entlang jedem Strahl beschreibt; und
des Mittelns der berechneten Eigenschaft für alle Strahlen innerhalb jedes VOIs zu einer Zahl, die den quantitativen Wert festlegt, der ein Indikator für den Grad der im Gehirn des Patienten bzw. Probanden vorliegenden neurodegenerativen Erkrankung ist.

2. Verfahren (200) nach Anspruch 1, wobei die Bilddaten ein Signal aus einer radioaktiven Tracersubstanz abbilden, die eine chemische Einheit umfasst, die selektiv an Amyloidprotein bindet, und die radioaktive Tracersubstanz zuvor dem Patienten bzw. Probanden verabreicht worden war.

3. Verfahren (200) nach Anspruch 1 oder Anspruch 2, das das Durchführen anatomischer Standardisierung an Amyloid-PET-Daten umfasst, wobei eine Bezugsvorlage aus einem Einzel-Patient/Proband-MRI-Scan im standardisierten Raum gewonnen wird und wobei wechselseitige Informationen oder normalisierte wechselseitige Informationen zwecks Optimierung verwendet werden.

4. Verfahren (200) nach Anspruch 3, wobei die Einzel-Patient/Proband-MRI-Bezugsvorlage mit einem anisotropen Filter unscharf gemacht wird, um Gewebegrenzen zu bewahren.

5. Verfahren nach Anspruch 1, wobei der Schritt des Analysierens der Bilddaten (240) weiterhin das Verwenden einer Wahrscheinlichkeitsmaske zum Festlegen von Bezugsregionen in einem durch die Bilddaten festgelegten Bild umfasst.

6. Verfahren (200) nach Anspruch 5, wobei die Wahrscheinlichkeitsmaske eine Graue-Substanz-Maske (540) ist.

7. Verfahren (200) nach Anspruch 6, wobei der Verwendungsschritt weiterhin den Schritt des Erstellens der Wahrscheinlichkeitsmaske für graue Substanz umfasst, wobei der Erstellungsschritt weiterhin die Schritte umfasst:
Koregistrieren von Bilddaten zwischen einem MRI-Scan und einem PET-Scan für eine Anzahl von Patienten bzw. Probanden;
Abgrenzen einer Bezugsregion in den koregistrierten MRI-Daten, wobei das Abgrenzen von einem Fachmann vorgenommen wird;
Umwandeln der Bilddaten zu einem standardisierten Raum; und
Berechnen einer Wahrscheinlichkeitskarte, die, für jedes Voxel, die Wahrscheinlichkeit zeigt, dass dieses Voxel in allen der Bezugsregionen der Patienten bzw. Probanden vorhanden ist.

8. Verfahren (200) nach Anspruch 7, wobei der Abgrenzungsschritt an zumindest einem von dem Cerebellum, dem Pons und der subkortikalen weißen Substanz angewandt wird.

9. Verfahren (200) nach Anspruch 5, das weiterhin den Schritt des Verwendens der Bezugsregionwahrscheinlichkeitsmaske umfasst, um an Daten Intensitätsnormalisierung vorzunehmen durch
Anwenden der Wahrscheinlichkeitsmaske auf die anatomisch standardisierten PET-Bilddaten;
Berechnen des mittleren Voxelwerts aller der durch die Maske festgelegten Voxel, wobei den Voxeln der relative Beitrag in Übereinstimmung mit der entsprechenden Wahrscheinlichkeit in der Maske gegeben wird; und
Teilen des gesamten Bildes durch das berechnete Mittel aus dem Berechnungsschritt.

10. Verfahren (200) nach Anspruch 2, das weiterhin den Schritt des Erhaltens eines Verhältnisbildes umfasst, wodurch eine Traceraufnahme bezüglich einer Bezugsregion skaliert wird.

11. Verfahren (200) nach Anspruch 10, wobei der quantitative Wert als Verhältnis von Substanzaufnahme in grauer Gehirnsubstanz zu Substanzaufnahme in weißer Gehirnsubstanz bestimmt wird, wobei die Substanz jede beliebige Substanz sein kann, deren Anteil sich verändert, wenn eine neurodegenerative Erkrankung vorliegt.

12. Verfahren (200) nach Anspruch 1, wobei der Schritt des Analysierens der Bilddaten (240) weiterhin die Schritte umfasst:
Anwenden eines VOI-Atlas auf das Verhältnisbild, wobei der VOI-Atlas Definitionen anatomischer Regionen beinhaltet, wobei der VOI-Atlas weiterhin Bereiche grauer Substanz und weißer Substanz der anatomischen Regionen definiert;
Berechnen der Aufnahme in der Graue-Substanz-Region in den vom VOI festgelegten Voxeln und der Graue-Substanz-Maske, und Berechnen, für die gleichen VOIs, der Aufnahme in der Weiße-Substanz-Region in den vom VOI festgelegten Voxeln und der Weiße-Substanz-Maske; und
Berechnen der Grau-Weiß-Verhältnisse für jedes VOI.

13. Verfahren (200) nach Anspruch 1, wobei der Berechnungsschritt weiterhin das Sampein der Bilddaten entlang dem Strahl umfasst, der von der Gehirnoberfläche ins Gehirn führt;
Analysieren des Intensitätsprofils und Extrahieren eines diagnostischen Merkmals, umfassend eins von dem Maximalgradienten, berechnet als Differenz zwischen zwei Punkten entlang dem Strahl in einem gewissen festen Abstand, und dem Verhältnis zwischen Punkten in einem gewissen Abstand entlang dem Strahl.

14. Verfahren (200) nach Anspruch 1, das weiterhin den Schritt des automatischen Auswählens einer anatomischen Standardisierung und/oder eines Bildanalysemodus in Abhängigkeit von der Klasse/den Klassen der Bilddaten umfasst.

15. Verfahren (200) nach einem der Ansprüche 1 bis 14, weiterhin umfassend das Berechnen eines Amyloidindex als gewichtetes Mittel aus den in einem Satz anatomischer Regionen berechneten Werten geteilt durch den entsprechenden Wert in zumindest einer Bezugsregion, wo der Wert in jeder Region mittels VOI-Analyse und/oder Intensitätsprofilanalyse berechnet wird.

16. Verfahren (200) nach einem der Ansprüche 1 bis 14, das weiterhin das Erzeugen einer dreidimensionalen Darstellung des Gehirns des Patienten bzw. Probanden umfasst, wobei die dreidimensionale Darstellung Farbcodierung von Regionen in Übereinstimmung mit der Aufnahme einer Substanz im Gehirn in jeweiligen dieser Regionen einschließt.

17. Verfahren (200) nach einem der Ansprüche 1 bis 14, das weiterhin das Erzeugen eines Berichts umfasst, wobei der Bericht eins oder mehrere beinhaltet von: einer Angabe des Vorliegens oder Nichtvorliegens neurodegenerativer Erkrankung beim Patienten bzw. Probanden; dem quantitativen Wert; einer quantitativen Angabe des Vorliegens oder Nichtvorliegens neurodegenerativer Erkrankung beim Patienten bzw. Probanden; Patienten- bzw. Probandeninformationen; Datum; Zeit; Bildern eines ursprünglichen Patienten- bzw. Probandenscans; verarbeiteten Bildern der Ergebnisse des Verfahrens nach einem der Ansprüche 1 bis 14; Tabellen mit Messungen; VOI-Ergebnissen; einem Amyloidindex; und einer Aussage darüber, ob jegliche Befunde innerhalb eines normalen Parameterbereichs liegen oder nicht.

18. Computerprogrammprodukt (144), das einen Computercode umfasst, der betreibbar ist, um eine Datenverarbeitungsvorrichtung (120) zu konfigurieren zwecks Implementierung eines oder mehrerer der Schritte des Verfahrens (200) nach einem der Ansprüche 1 bis 17.

19. Computerprogrammprodukt (144) nach Anspruch 18, das auf einem Trägermedium (142) vorgesehen ist.

20. Computerprogrammprodukt (144) nach Anspruch 19, wobei das Trägermedium (142) eins oder mehrere umfasst von: einer Magnetplatte, einem Magnetband, einer optischen Platte, einem elektronischen Signal, einem optischen Signal, einem Funksignal und einer Halbleitervorrichtung.

21. System (100) zur klinischen Evaluierung einer bei einem Patienten bzw. Probanden vorliegenden neurodegenerativen Erkrankung, wobei das System umfasst:
ein PET-Bildbeschaffungsmodul (122), das betreibbar ist, um Bilddaten zu beschaffen, die repräsentativ für ein Gehirn eines Patienten bzw. Probanden sind; und
eine Bildanalysevorrichtung (124), wobei die Bildanalysevorrichtung betreibbar ist, um das Verfahren nach Anspruch 1 durchzuführen.

22. System (100) nach Anspruch 19, wobei die Bildanalysevorrichtung (124) weiterhin betreibbar ist, um den quantitativen Wert aus den Bilddaten zu bestimmen, durch Bestimmen der Konzentration von Amyloidplaques im Gehirn des Patienten bzw. Probanden.

23. System (100) nach einem der Ansprüche 21 und 22, wobei die Bildanalysevorrichtung (124) weiterhin betreibbar ist, um in einem von den Bilddaten festgelegten Bild eine Bezugsregion festzulegen, aus der der Grad der beim Patienten bzw. Probanden vorliegenden neurodegenerativen Erkrankung bestimmt werden kann.

24. System (100) nach einem der Ansprüche 21 bis 23, wobei die Bildanalysevorrichtung (124) weiterhin betreibbar ist, um den quantitativen Wert als Verhältnis von Substanzaufnahme in grauer Gehirnsubstanz zu Substanzaufnahme in weißer Gehirnsubstanz zu bestimmen.

25. System (100) nach einem der Ansprüche 21 bis 24, wobei die Bildanalysevorrichtung (124) weiterhin betreibbar ist, um den quantitativen Wert als Veränderungsrate der Bilddatenintensität entlang einer vorgegebenen Projektion im Gehirn zu bestimmen.

26. System (100) nach einem der Ansprüche 21 bis 25, wobei die Bildanalysevorrichtung (124) weiterhin betreibbar ist, um automatisch eine anatomische Standardisierung und/oder einen Bildanalysemodus in Abhängigkeit von der Klasse/den Klassen der Bilddaten auszuwählen.

27. System (100) nach einem der Ansprüche 21 bis 26, das weiterhin eine graphische Benutzerschnittstelle (GUI) (123) umfasst, die betreibbar ist, um eine dreidimensionale Darstellung des Gehirns des Patienten bzw. Probanden zu erzeugen, wobei die dreidimensionale Darstellung Farbcodierung von Regionen in Übereinstimmung mit der Aufnahme einer Substanz im Gehirn in jeweiligen dieser Regionen einschließt.

28. System (100) nach Anspruch 27, wobei die GUI (123) weiterhin betreibbar ist, um Daten in tabellarischer Form mit der dreidimensionalen Darstellung zu verknüpfen.

## Revendications

1. Procédé de tomographie par émission de positron ("PET") (200) destiné à faciliter l'évaluation clinique d'une maladie neuro-dégénérative présente chez un sujet, le procédé comprenant :
l'acquisition d'une image de TEP (220) représentative d'un cerveau d'un sujet ; et
l'analyse des données d'image (240) afin de déterminer une valeur quantitative (260) à partir des données d'image, la valeur quantitative étant représentative d'un niveau de maladie neuro-dégénérative présente dans le cerveau du sujet, qui est déterminée par la concentration en amyloïde dans le cerveau du sujet ;
dans lequel un procédé de standardisation anatomique est mis en oeuvre afin de transformer les données d'image dans un espace anatomique standardisé en utilisant un procédé en deux étapes, le procédé en deux étapes comprenant :
l'exécution d'un recalage global des données d'image sur un gabarit de référence ; et
l'affinage du recalage en utilisant un recalage rigide dans une zone bordée par une forme en 3D interne et externe, dans lequel le recalage final est une association des recalages global et rigide et dans lequel les données d'image entre la forme 3D interne et externe sont interpolées afin de produire une transition douce entre la zone affinée localement et des données de recalage global ;
le procédé de TEP comprenant, en outre, les étapes de
définition d'un certain nombre de points de surface et de rayons suivant la surface du cerveau normal dans l'espace anatomique standardisé, et d'une étiquette définissant à quel volume d'intérêt ("VOI") dans un recueil de VOI chaque point de surface appartient ;
calcul d'un profil d'intensité pour chacun des VOI définis par l'étape de définition en utilisant des traces perpendiculaires à la surface du cerveau à partir de données d'acquisition qui ont été à la fois standardisées de manière anatomique et normalisées en intensité, dans lequel la normalisation d'intensité calibre les données en fonction de l'assimilation dans une région qui est supposée ne pas être affectée par la maladie neuro-dégénérative ;
calcul informatique d'une propriété décrivant le profil d'intensité le long de chaque rayon ; et
calcul de la moyenne de la propriété calculée de manière informatique pour tous les rayons à l'intérieur de chaque VOI en un nombre définissant la valeur quantitative représentative du niveau de maladie neuro-dégénérative présente dans le cerveau du sujet.

2. Procédé (200) selon la revendication 1, dans lequel les données d'image représentent un signal à partir d'une substance formant traceur radioactif comprenant une entité chimique qui se lie de manière sélective à la protéine amyloïde, et ladite substance formant traceur radioactif a été préalablement administrée audit sujet.

3. Procédé (200) selon la revendication 1 ou 2, comprenant l'exécution d'une standardisation anatomique sur des données de TEP d'amyloïde, dans lequel un gabarit de référence est obtenu à partir de données d'acquisition par IRM sur un seul sujet dans un espace standardisé et dans lequel des informations mutuelles ou des informations mutuelles standardisées sont utilisées lors de l'optimisation.

4. Procédé (200) selon la revendication 3, dans lequel le gabarit de référence d'IRM sur un seul sujet est estompé avec un filtre anisotrope dans le but de préserver les frontières de tissu.

5. Procédé selon la revendication 1, dans lequel l'étape d'analyse des données d'image (240) comprend, en outre, l'utilisation d'un masque de probabilité afin de définir des zones de référence sur une image définie par les données d'image.

6. Procédé (200) selon la revendication 5, dans lequel ledit masque de probabilité est un masque de matière grise (540).

7. Procédé (200) selon la revendication 6, dans lequel ladite étape d'utilisation comprend, en outre, l'étape de création dudit masque de matière grise de probabilité, ladite étape de création comprend, en outre, les étapes de :
recalage simultané des données d'image entre des données d'acquisition par IRM et des données d'acquisition par TEP pour un certain nombre de sujets ;
tracé d'une zone de référence sur les données d'IRM recalées simultanément, dans lequel ledit tracé est mis en oeuvre par un expert ;
transformation des données d'image dans un espace standardisé ; et
calcul informatique d'une carte de probabilité qui, pour chaque voxel, montre la probabilité que ce voxel soit présent dans toutes les zones de référence des sujets.

8. Procédé (200) selon la revendication 7, dans lequel ladite étape de tracé est appliquée sur au moins l'un du cervelet, de la protubérance cérébrale, et de la matière blanche sub-corticale.

9. Procédé (200) selon la revendication 5, comprenant, en outre, l'étape d'utilisation du masque de probabilité de zone de référence afin de normaliser les données en intensité par :
application du masque de probabilité sur les données d'image anatomiques standardisées ;
calcul informatique de la valeur moyenne de voxel de tous les voxels définis par le masque, dans lequel les voxels sont affectés de la contribution relative en fonction de la probabilité correspondante sur le masque ; et
division de la totalité de l'image avec la moyenne calculée à partir de l'étape de calcul informatique.

10. Procédé (200) selon la revendication 2, comprenant, en outre, l'étape d'obtention d'une image de rapport de telle sorte qu'une assimilation de traceur est calibrée relativement à une région de référence.

11. Procédé (200) selon la revendication 10, dans lequel la valeur quantitative est déterminée sous la forme d'un rapport d'assimilation de substance par la matière grise du cerveau sur l'assimilation de substance par la matière blanche du cerveau, dans lequel le rapport de la substance change lorsque la maladie neuro-dégénérative est présente.

12. Procédé (200) selon la revendication 1, dans lequel l'étape d'analyse des données d'image (240) comprend, en outre, les étapes de :
application d'un recueil de VOI sur l'image de rapport, dans lequel le recueil de VOI comporte des définitions de régions anatomiques, dans lequel ledit recueil de VOI définit, en outre, des zones de matière grise et de matière blanches des régions anatomiques ;
calcul informatique de l'assimilation dans la région de matière de grise sur les voxels définis par le VOI et le masque de matière grise, et calcul pour le même VOI ; de l'assimilation dans la région de matière blanche sur les voxels définis par le VOI et le masque de matière blanche ; et
calcul des rapports grise/blanche pour chaque VOI.

13. Procédé (200) selon la revendication 1, dans lequel l'étape de calcul, comprend, en outre, l'échantillonnage des données d'image le long du rayon partant de la surface du cerveau vers le cerveau ;
l'analyse du profil d'intensité et l'extraction d'une particularité diagnostique comprenant l'un du gradient maximum calculé sous la forme d'une différence entre deux points le long du rayon à une certaine distance fixe et du rapport entre des points à une certaine distance le long du rayon.

14. Procédé (200) selon la revendication 1, comprenant, en outre, l'étape de sélection automatique d'un mode de standardisation anatomique et/ou d'analyse d'image en fonction de la classe/des classes des données d'image.

15. Procédé (200) selon l'une quelconque des revendications 1 à 14, comprenant, en outre, le calcul d'un indice d'amyloïde sous la forme d'une moyenne pondérée des valeurs calculées à l'intérieur d'un ensemble de régions anatomiques divisée par la valeur correspondante dans au moins une région de référence, dans lequel la valeur dans chaque région est calculée en utilisant l'analyse de VOI et/ou l'analyse de profil d'intensité.

16. Procédé (200) selon l'une quelconque des revendications 1 à 14, comprenant, en outre, la production d'une représentation tridimensionnelle du cerveau du sujet, dans lequel la représentation tridimensionnelle comporte le codage de couleur de régions en fonction de l'assimilation d'une substance dans le cerveau dans certaines de ces régions respectives.

17. Procédé (200) selon l'une quelconque des revendications 1 à 14, comprenant, en outre, la production d'un rapport, le rapport comportant un ou plusieurs parmi : une indication de la présence ou de l'absence de la maladie neuro-dégénérative chez le sujet ; la valeur quantitative ; une indication quantitative de la présence ou de l'absence de la maladie neuro-dégénérative chez le sujet ; des informations sur le patient ; la date ; l'heure ; des images d'une opération d'imagerie de patient d'origine ; des images traitées des résultats du procédé selon l'une quelconque des revendications 1 à 14 ; des tables avec des mesures ; des résultats de VOI ; un indice d'amyloïde ; et un bilan précisant si les constatations se situent ou non à l'intérieur d'une plage de paramètre normale.

18. Produit formant programme informatique (144) comprenant un code informatique pouvant servir à configurer un dispositif de traitement numérique (120) destiné à mettre en oeuvre une ou plusieurs des étapes du procédé (200) selon l'une quelconque des revendications 1 à 17.

19. Produit formant programme informatique (144) selon la revendication 18, fourni sur un support d'enregistrement (142).

20. Produit formant programme informatique (144) selon la revendication 19, dans lequel le support d'enregistrement (142) comprend un ou plusieurs parmi un disque magnétique, une bande magnétique, un disque optique, un signal électronique, un signal optique, un signal radio, et un dispositif à semiconducteur.

21. Dispositif (100) destiné à assurer l'évaluation clinique d'une maladie neuro-dégénérative présente chez un sujet, le dispositif comprenant :
un module d'acquisition d'image de TEP (122) pouvant servir à acquérir des données d'image représentatives d'un cerveau d'un sujet ; et
un analyseur d'image (124), dans lequel l'analyseur d'image peut être utilisé afin de mettre en oeuvre le procédé selon la revendication 1.

22. Dispositif (100) selon la revendication 19, dans lequel l'analyseur d'image (124) peut, en outre, servir à déterminer la valeur quantitative à partir des données d'image en déterminant la concentration en plaques amyloïdes dans le cerveau du sujet.

23. Dispositif (100) selon l'une quelconque des revendications 21 et 22, dans lequel l'analyseur d'image (124) peut, en outre, être utilisé afin de définir une région de référence sur une image définie par les données d'image à partir desquelles on peut déterminer le niveau de la maladie neuro-dégénérative présente chez le sujet.

24. Dispositif (100) selon l'une quelconque des revendications 21 à 23, dans lequel l'analyseur d'image (124) peut, en outre, servir à déterminer la valeur quantitative sous la forme d'un rapport d'une assimilation de substance dans de la matière grise du cerveau sur l'assimilation de substance dans la matière blanche du cerveau.

25. Dispositif (100) selon l'une quelconque des revendications 21 à 24, dans lequel l'analyseur d'image (124) peut, en outre, servir à déterminer la valeur quantitative sous la forme d'un taux de variation sur l'intensité de données d'image suivant une projection prédéterminée dans le cerveau.

26. Dispositif (100) selon l'une quelconque des revendications 21 à 25, dans lequel l'analyseur d'image (124) peut, en outre, servir à sélectionner de manière automatique un mode de standardisation anatomique et/ou d'analyse d'image en fonction de la classe/des classes des données d'image.

27. Dispositif (100) selon l'une quelconque des revendications 21 à 26, comprenant, en outre, une interface graphique d'utilisateur (GUI) (123) pouvant être utilisée afin de produire une représentation tridimensionnelle du cerveau du sujet, dans lequel la représentation tridimensionnelle comporte le codage en couleur de régions en fonction de l'assimilation d'une substance dans le cerveau dans ces régions respectives.

28. Dispositif (100) selon la revendication 27, dans lequel l'interface GUI (123) peut, en outre, servir à lier des données sous forme tabulaire avec la représentation tridimensionnelle.
